# EUROPEAN PATENT APPLICATION

(11) **EP 2 728 016 A1**
(43) Date of publication of application: **07.05.2014**
(21) Application number: 12190958.4
(22) Date of filing: 01.11.2012
(51) Int. Cl.: C12Q 1/68

(54) **Long hair phenotype in canidae**

(71) Applicant: Stiftung Tierärztliche Hochschule Hannover, 30559 Hannover (DE)
(72) Inventor: Distl, Ottmar, 30559 Hannover (DE); Dierks, Claudia, 31319 Sehnde (DE); Mömke, Stefanie, 30175 Hannover (DE); Philipp, Ute, 30163 Hannover (DE)
(74) Representative: Kröncke, Rolf

(57) **Abstract**

The present invention provides a method for the analysis of the genetic disposition of long hair phenotype in canidae based on determining physically specific mutations in at least one allele of the gene FGF5. In addition, the present invention relates to the use of said new mutations described herein for determining a genetic disposition for a long hair phenotype in a canidae. The canidae are particular selected from Akita, Samoyed, Afghan hound, Eurasier and Siberian husky. Moreover, a genetic marker as well as test systems or test kits based on said genetic marker for use in determining genetic disposition of a canidae for long hair phenotype is provided. Finally, isolated nucleic acid molecules and oligonucleotide or probes are provided comprising the mutations defined herein.

## Description

The present invention provides a method for the analysis of the genetic disposition of long hair phenotype in canidae based on determining physically specific mutations in at least one allele of the gene *FGF5.* In addition, the present invention relates to the use of said new mutations described herein for determining a genetic disposition for a long hair phenotype in a canidae. The canidae are particular selected from Akita, Samoyed, Afghan hound, Eurasier and Siberian husky. Moreover, a genetic marker as well as test systems or test kits based on said genetic marker for use in determining genetic disposition of a canidae for long hair phenotype is provided. Finally, isolated nucleic acid molecules and oligonucleotide or probes are provided comprising the mutations defined herein.

### Prior Art

Hair length is an important morphological trait among most dog breeds. The breed standard often allows only one specific hair length for the majority of registered dog breeds. Although long hair is inherited as a simple recessive trait in different dog breeds, the length of long hair is variable depending on the breed, see e.g. Housley & D. J. Venta, 2006, Animal Genetics, 37, 309 - 315. At present, the only mutant gene found that leads to the long hair phenotype in mammalian species is the *fibroblast growth factor 5 (FGF5)* (e.g. Cadieu et al., 2009, Science 326, 150 - 153). A *FGF5:p.Cys95Phe* mutation was completely concordant with the long hair phenotype in many dog breeds, see Housley & Venta, 2006 or Cadieu et al., 2009, but not in dogs like the Afghan hound. Other mutations have been found in the *FGF5* gene like the *FGF5:g.145_150dupACCAGC* in exon 1 leading to a duplication of Thr and Ser (TS) which was found in high linkage disequilibrium with the *FGF5:p.Cys95Phe* polymorphism but had no influence on hair length (Housley & Venta 2006). A genome-wide association study in dogs gave no hints on gene loci other than the *FGF5* locus on dog chromosome 32 associated with the long hair phenotype. So far no other mutation in the *FGF5* gene has been described influencing the hair length.

Alternative splicing of *FGF5* results in the expression of two mRNAs in mammals, in one of which exon 2 is absent (FGF5 short form, FGF5S) (see e.g. Housley & Venta 2006). It has been suggested that the two resulting protein variants function together to regulate the hair cycle. Also from other mammals, like mice and cats, the importance of the *FGF5* in long hair phenotype is known.

Since at present, the only mutant gene found that leads to the long hair phenotype in mammalian species is the *FGF5:p.Cys95Phe (FGF5:c.284G>T)* mutation which contributes to the long hair phenotype in many but not all dog breeds, the objective of the present invention was to provide new processes and means for the analysis of the genetic disposition of long hair phenotype in canidae, in particular, with respect to dog breeds for which mutations for long hair phenotype had not been described yet.

In another aspect, the present invention aims in providing suitable test kits and test systems for use in determining genetic disposition of long hair phenotype in canidae. Finally, new isolated nucleic acid molecules including isolated oligonucleotide or probes comprising the newly identified mutations are provided.

### Summary of the present invention

The present invention provides in a first aspect a process for the analysis of the genetic disposition of long hair phenotype in canidae wherein in at least one allele of the gene *FGF5* at least one of the mutations consisting of: *FGF5:c.578C>T, FGF5:c.559_560dupGG, FGF5:g.8193T>A* or *FGF5:c.556_571del16* is determined.

In particular, the process is suitable for determining the genetic disposition in a canidae selected from Akita, Samoyed, Afghan hound, Eurasier and Siberian husky.

In another aspect, the present invention provides genetic marker for use in determining genetic disposition of canidae for a long hair phenotype by a process according to the present invention and the use of the mutations for determining a genetic disposition for long hair phenotype in a canidae, in particular, in the species selected from Akita, Samoyed, Afghan hound, Eurasier and Siberian husky.

Moreover, test systems or test kits for use in determining the genetic disposition of long hair phenotype in canidae are provided. Said test system or test kit comprise means for determining the presence of at least one mutation in at least one allele of the gene *FGF5,* said mutations are selected from the mutations consisting of *FGF5:c.578C>T, FGF5:c.559_560dupGG, FGF5:g.8193T>A* or *FGF5:c.556_571del16* and instructions for use that means in a process according to the present invention.

Finally, the present invention provides isolated nucleic acid molecules comprising the mutations according to present invention as well as isolated oligonucleotide or probes comprising a nucleic acid sequence of the gene *FGF5* having the mutation according to present invention.

### Brief description of the drawings

Fig.1: In figure 1, the exon-intron structure of the canine *FGF5* (isoform 1) is shown. All mutations in *FGF5* are displayed. The two mutations in exon 1 were previously published. The four novel mutations identified in our study were located in intron 1 and exon 3 (marked in bold).
Fig.2: In figure 2, the *FGF5:g.8193T>A* mutation in Afghan hound is determined based on mismatch-PCR-RFLP.
Fig.3: In figure 3, the genotyping results for the *FGF5:c.556_571del16* mutation in Eurasier is shown based on gel electrophoresis.
Fig.4: In figure 4, the genotyping results for the *FGF5:c.559_560dupGG* mutation in Afghan hound is shown based on gel electrophoresis.
Fig.5: In figure 5, the *FGF5:c.578C>T* mutation in Akita is determined based on mismatch-PCR-RFLP.

### Detailed description of the present invention

In a first aspect, the present invention provides a process for the analysis of the disposition of long hair phenotype in canidae wherein at least one allele of the *FGF5* at least one of the mutations consisting of *FGF5:c.578C>T, FGF5:c.559_560dupGG, FGF5:g.8193T>A* or *FGF5:c.556_571del16* is determined.

As used herein, the term "analysis of a genetic disposition" or "determining genetic disposition" identifies that the DNA of the canidae is obtained, sequenced and the nucleic acid sequence is determined. Determining the mutation identifies that based on the physical analysis of the sequence, the mutation is identified.

As used herein, the term "mutation" and "abberation" are used interchangeably and have the same meaning unless otherwise indicated. The term "comprise" or "comprising" or the term "contain" or "containing" include the specific embodiment of "consist" or "consisting".

As used herein, the term genetic marker identifies a DNA sequence with a known location on a chromosome that can be used to identify individuals or species. Typically, a genetic marker is a short DNA sequence, such as a sequence surrounding a single base pair exchange (SNP) or other type of mutations or alterations.

The present invention aims in providing four newly detected mutations including one deletion, one duplication and two nucleotide polymorphism (SNPs) causing long hair phenotype in canidae. While some of these mutations were observed to cause the long hair phenotype if homozygous, all mutations caused long hair phenotype when compound heterozygous with another mutation for long hair. All observed combinations of mutations in *FGF5* which cause the long hair phenotype as determined in the examples are identified in table 1.

**Table 1. Recessive mutations in the FGF5 gene associated with longhair phenotypes in dogs. The FGF5:c.578C>T and the FGF5:c.559_560dupGG mutations were also observed homozygous in longhaired dogs. The mutation indicated by an asterics (*) are novel ones.**

| Mutation 1 | Mutation 2 | phenotype | Breed |
|---|---|---|---|
| *FGF5:c.284G>T* | *FGF5:g.8193T>A** | longhair | Afghan hound |
| *FGF5:c.284G>T* | *FGF5:c.559_560dupGG** | longhair | Afghan hound, Eurasier |
| *FGF5;c.559_560 dupGG** | *FGF5:c.559_560dupGG** | longhair | Afghan hound |
| *FGF5:c.284G>T* | *FGF5;c.556_571del16** | longhair | Eurasier |
| *FGF5:c.284G>T* | *FGF5:c.578C>T** | longhair | Samoyed |
| *FGF5:c.578C>T** | *FGF5:c.578C>T** | longhair | Akita, Samoyed |

At least one of the mutations described herein may be used, optionally together with the known marker *FGF5:p.Cys95Phe (FGF5:c.284G>T).* The test system comprising at least one of the markers identified herein optionally in combination with the known marker identified above, may be used to test for carry of long hair in dogs. These tests can be performed in puppies and, thus, are suitable to differentiate between long hair dogs in short hair dog breeds. Thus, the test system or test kit according to the present invention as well as the process and the genetic marker described herein including the isolated oligonucleotide or probe or the isolated nucleic acid molecule according to the present invention are particularly useful for breeding because the carrier status of an allele for long hair can be determined before a selection decision is made. Up to now, there is no other method or diagnostic test available which would detect these four novel mutations for long hair in dogs.

It is preferred, that at least the mutation *FGF5:c.578C>T* is determined in the process according to the present invention. In particular with respect to the long hair phenotype in Akita and Samoyed, said mutation is of particular interest. The *FGF5:c.578C>T* mutation may be present homozygously or heterozy-gously in the animals.

In another preferred embodiment, the mutations are present on both alleles. Alternatively, the animals are characterized in having one of the referenced mutations on one allele while having a second mutation on the other allele. As shown in table 1 long hair phenotype in the canidae may occur homozygously or the mutation may occur compound heterozygous with another mutation for long hair. In a preferred embodiment, the genetic disposition is determined in a canidae selected from Akita, Samoyed, Afghan hound, Eurasier and Siberian husky. In particular, it is a preferred embodiment to determine mutation *FGF5:c.578C>T* in Akita, Samoyed and Siberian husky. The presence of said mutation is indicative for a genetic disposition of long hair phenotype.

The mutation identified according to the present invention may be determined on the coding strand or the non-coding strand, respectively.

The process according to the present invention is particularly useful for selecting individual canidae for breeding purposes. As identified above, it is possible to determine the hair genotype at a very early stage, thus, the selection decision can be made very early.

The skilled person is well aware of suitable methods for analyzing the genetic disposition, as of suitable methods for determining the presence or absence of the mutations identified herein. Suitable methods encompass common a process of molecular biology including PCR based methods or sequencing based methods. Alternatively, restriction analysis may be performed, e.g. RFLP. It is preferred that the analysis is done by amplification of genomic DNA or cDNA and, subsequently, sequencing based on common sequence technologies.

Suitable primer pairs for amplification and sequencing can be obtained by applying common techniques for determining the same including commercially available programs for designing suitable primer pairs.

In a further aspect, the present invention relates to the use of at least one of the mutations of *FGF5:c.578C>T, FGF5:c.559_560dupGG, FGF5:g.8193T>A* or *FGF5:c.556_571del16* for determining a genetic disposition for a long hair phenotype in a canidae.

In particular, the present invention provides new genetic markers for use in determining genetic disposition of canidae for a long hair phenotype. Said genetic markers may be determined by a process according to the present invention. The genetic markers are *FGF5:c.578C>T, FGF5:c.559_560dupGG, FGF5:g.8193T>A* or *FGF5:c.556_571del16.*

The material or sample for the analysis of the genetic disposition is obtained of canidae by suitable means. Said sample may be an EDTA blood, hair root, semen or tissue sample containing DNA from said canidae. The DNA is obtained by suitable methods and is processed further allowing to determining the presence or absence of the mutations as identified herein.

DNA is extracted and analysis of the DNA is conducted by suitable methods for amplification and sequencing of the *FGF5* gene.

In particular, genetic disposition of long hair phenotype in Akita and Samoyed is given at least in the presence of mutation *FGF5:c.578C>T,* optionally in combination with the presence of the mutation *FGF5:c.284G>T.* Moreover, long hair genetic disposition with long hair phenotype may be found in Eurasier when a mutation *FGF5:c. 556_571del16* and/or *FGF5:c.284G>T* is present or, alternatively, the mutation *FGF5:c.559_560dupGG* in combination with *FGF5:c.284G>T.* The same holds true for the Afghan hound for the mutation *FGF5:c.559_560dupGG* which may be present homozygously or in combination with *FGF5.c.284G>T.* In addition a long hair phenotype in Afghan hound is associated with the mutation *FGF5:5.8193T>A* obviously in combination with *FGF5:c.284G>T or FGF5:c.559_560dupGG.*

Moreover, a test system or test kit for use in determining the genetic disposition of long hair phenotype canidae is provided comprising means for determining the presence of at least one mutation at least one allele of the gene *FGF5,* said mutations are selected from the group consisting of *FGF5:c.578C>T, FGF5:c.559_560dupGG, FGF5:g.8193T>A* or *FGF5:c.556_571del16* and instructions for use of that means in a process according to the present invention. Typically, said test kit or test system comprises primer pair allowing determination of the mutation specified herein. Suitable primers are identified e.g. in table 2. In preferred embodiments, the mutation *FGF5:g.8193T>A* is determined using at least one of the primers of SEQ ID Nos. 3 and 4, the mutation *FGF:c.556_571del16* is determined using at least one of the primer set of SEQ ID Nos. 5 and 6. Moreover, the mutation *FGF5:c.559_560dupGG* is identified using at least one of the primer pair of SEQ ID Nos. 7 and 8, while the mutation *FGF5:c.578C>T* is determined using at least one of the primers of SEQ ID Nos. 9 and 10.

The specific primer pairs, the mutation the FGF5 gene for long hair phenotypes in dogs as well as breeds in which the mutation was observed are provided in table 2.

**Table 2. Mutations in the FGF5 gene for longhair phenotypes in dogs, their effect on the protein, breeds in which the mutation was observed, genotyping method, primer, product size (P) in base pairs and annealing temperature (AT). The mutation FGF5:c.284G>T was previously published by Hously and Venta (2006) and determined using at least one of the primer of Seq. ID. Nos. 1 and 2. The aberrations marked in bold are novel ones.**

| Mutation | Effects on FGF5 | Breeds | Genotyping method | Primer (5'->3') | P | AT |
|---|---|---|---|---|---|---|
| | | | | **():Seq ID. No.** | | |
| *FGF5:c.284G >T* | p.Cys95Phe | several | PCR-RFLP, *PstI* and Cac8I | F: CCTCTTCTTCCTCCG TCTCC(1) | 279 | 58 |
| | | | | R: CAGGGTGCAAAACAACC (2) | | |
| *FGF5:g.8193 T>A* | prevents splicing of exon 2 | Afghan hound | Mismatch-PCR-RFLP, *BglII* | F: AGAGGAGTCTGTGTTTT ATTTTGGGAGATC (3) | 270 | 60 |
| | | | | R:CCCAAGAAATCATTAAGT CCTCTG (4) | | |
| FGF5;c.556_ *571del16* | p.Ala186Thr fsTerm69 | Eurasier | Gel electrophoresis (LI-COR4300), DY682 | F.DY682-GTTCAGGGAGC GATTTCAAG (5) | 344 /36 | 60 |
| | | | | R:GCAAGGCATGGTTTCTCA C (6) | 0 | |
| FGF5;c.559_ *560dupGG* | p.Arg188Ala fsTerm73 | Afghan hound, Eurasier | Gel electrophoresis (LI-COR4300), DY682 | F.DY682-GTTCAGGGAG CGATTTCAAG (7) | 360 /36 | 60 |
| | | | | R:GCAAGGCATGGTTTCTCA C (8) | 2 | |
| *FGF5:c.578C >T* | p.Ala193Val | Akita, Samoyed, Sibirian husky | *RFLP, HhaI* | F:GTTCAGGGAGCGA TTTCAAG (9) | 358 | 58 |
| | | | | R:AAGGCATGGTTTCTC ACCAG (10) | | |

Moreover, the present invention provides newly isolated nucleic acid molecules comprising a mutation within the gene *FGF5* selected from *FGF5:c.578C>T, FGF5:c.559_560dupGG, FGF5:g.8193T>A* or *FGF5:c.556_571del16.*

Finally, isolated oligonucleotides or probes comprising the nucleic acid sequence of the gene *FGF5* having a mutation of the group consisting of *FGF5:c.578C>T, FGF5:c.559_560dupGG, FGF5:g.8193T>A* or *FGF5:c.556_571del16* are provided.

Spontaneous mutations in other animals within the *FGF5* gene associated with the long hair phenotype are shown in table 3 including species, location, mode of inheritance and reference.

**Table 3. Spontaneous mutations within the FGF5 gene in mammalian species associated with the long hair phenotype including species, location, mode of inheritance and reference.**

| Species | Mutation | Location | Inheritance | Reference |
|---|---|---|---|---|
| Cat | *FGF5:c.356insT* | exon 1 | recessive | Kehler *et al.* 2007 |
| Cat | *FGF5:c.406C>T* | exon 2 | recessive | Kehler *et al.* 2007 |
| Cat | *FGF5:c.474delT* | exon 3 | recessive | Kehler *et al.* 2007 |
| Cat | *FGF5:c.475A>C* | exon 3 | recessive | Kehler *et al.* 2007 |
| Dog | *FGF5:c.284G>T* | exon 1 | recessive | Hously and Venta 2006 |
| Mouse *(FGF5^{go})* | Deletion including exon 1 | exon 1 | recessive | Sundberg *et al.* 1997 |
| Mouse *(FGF5^{go-Utr})* | Retrotransposon mediated deletion | exon 3 | recessive | Mizuno *et al.* 2011 |

Kehler J.S., et. Al., (2007), Journal of Heredity 98, 555-66;
Sundberg J.P., et. al., (1997), Veterinary Pathology 34:171-9.
Mizuno S., et.al, (2011), Experimental Animals 60, 161-7.

The invention will be described by a way of examples further without limiting the same thereto.

### Examples

### Materials and methods

### Animals, DNA and RNA isolation

For mutation screening of the *FGF5* gene, we collected EDTA-blood samples and hair root samples of three shorthaired and three longhaired Akitas and three Afghan hounds, all longhaired for *FGF5* sequence analyses. EDTA-blood samples of these dogs and two Eurasiers were used for genomic *FGF5* analyses. For genotyping we used a total of 84 samples from the breeds Afghan hound, Akita, Eurasier, Samoyed and Siberian husky. RNA was isolated from hair root samples using the RNeasy Lipid Tissue Mini Kit (Qiagen, Hilden, Germany). Aliquots of about 200 ng total RNA were reverse transcribed into cDNA using Maxima® First Strand cDNA Synthesis Kit (Fermentas, St. Leon-Rot, Germany). The DNA was isolated from EDTA-blood samples or hair roots using the QIAamp 96 Spin Blood Kit (Qiagen) or by ethanol precipitation.

### Sequencing the FGF5 Gene

Primer pairs for the amplification of the cDNA and genomic DNA of the coding sequence of the canine *FGF5* were designed using the *canis lupus familiaris* reference mRNA and genomic sequence of CFA32, CanFam3.1 (NM_001048129 and NC_006614) and Primer3 (http://frodo.wi.mit.edu/cgi-bin/primer3/primer3_www.cgi) after masking repetitive sequences using the RepeatMasker (http://repeatmasker.genome.washington.edu/) (Primers are shown in table 2). PCR reactions were performed in 22-µl reaction volumes using 10-20 ng DNA, 2.2µl 10x incubation buffer containing 15 mM MgCl₂, 4.4 µl 5x PCR enhancer (Peqlab, Erlangen, Germany), 0.4 µl 10 mM dNTP, 10 pmol of each primer and 0.2 µl *Taq* polymerase (5 U/µl, Qbiogene, Heidelberg, Germany). The PCR amplification was carried out using the following standard program with variable annealing temperature between 59 and 60°C: 94 °C for 4 min, followed by 36 cycles at 94 °C for 30 s, optimum annealing temperature for 30 s, 72 °C for 30 s per 500 bp, 72 °C for 5 min and finally storing at 4 °C for 10 min. The bands of the two transcripts of *FGF5* were collected from a 0.6 % agarose gel and the cDNA was extracted using the QIAEX II Gel Extraction Kit (Qiagen). Sequences were generated using internal sequencing primer pairs and the Applied Biosystems 3500 Genetic Analyzer (Life Technologies, Darmstadt, Germany). For analyzing the sequences, we used Sequencher 4.8 (GeneCodes, Ann Arbor, Michigan, USA).

### Genotyping

For genotyping the *FGF5:pCys95Phe* mutation a PCR-RFLP was performed (Housley & Venta 2006). For the novel mutations identified in intron 1 and exon 3 primer pairs were designed in the same way as described above. PCR reactions were performed in 12-µl reaction volumes using 10-20 ng DNA, 1.2µl 10x incubation buffer containing 15 mM MgCl₂, 2.4 µl 5x PCR enhancer (Peqlab), 0.2 µl 10 mM dNTP, 5 pmol of each primer and 0.1 µl *Taq* polymerase (5 U/µl, Qbiogene). The PCR amplification was carried out using the following standard program with variable annealing temperature between 58 and 60°C: 94 °C for 4 min, followed by 36 cycles at 94 °C for 30 s, optimum annealing temperature for 30 s, 72 °C for 30 s, 72 °C for 5 min and finally storing at 4 °C for 10 min. Genotyping was performed via restriction fragment length polymorphisms (RFLP). Using mismatch priming PCR-RFLP technique according to Quadros L, et al., (2008) Haemophilia 14: 628-9 a *BglII* restriction site was created in the amplicons, which had the *FGF5:g.8193T>A* mutation in intron 1. RFLP was done in 10 µl reaction volumes using 1 µl buffer, if required 0.1 µl bovine serum albumin (BSA) and 0.1 µl of the specific restriction enzyme, 5 to 8 µl of the PCR product. After digestion over night, the fragments were separated by gel electrophoresis using 2 to 3.5% mo-sieve agarose gels and evaluated by visual examination. For genotyping the deletion and duplication polymorphisms we performed a PCR with DY682-labelled primers. The products were size-fractionated by gel electrophoresis on an automated LI-COR 4300 sequencer (LI-COR, Lincoln, NE, USA) using 5 % polyacrylamide denaturating gels (Rotiphorese Gel40, Carl Roth, Karlsruhe, Germany). Allele sizes were detected using a DY682-labelled DNA ladder, and the genotypes were assigned by visual examination.

### Bioinformatic analyses

Open reading frames of the mutated *FGF5* mRNAs were determined using ORF finder (http://www.bioinformatics.org/sms/orf_find.html). The predicted proteins were aligned to the known FGF5 proteins. GenBank numbers assigned to the canine *FGF5* cDNAs are NP_001041594 and DQ268833, including the transcript variant without exon 2. We aligned several canine *FGF5* long variants with other species as follows: cat (NP_001108018), human isoform 1 (NP_004455), mouse (NP_034333) and rat (NP_071547). We aligned several canine FGF5 short variants (isoform 2) with other mammalian FGF5 short variants as follows: cat (CAL81527), human (NP_149134), mouse (BAA33737) and rat (BAA10967). Protein alignment was done using EBI toolbox ClustalW (http://www.ebi.ac.uk/Tools/ msa/clustalw2/). The predicted effect of the mutations found in this study on protein structure and function was analysed with Polyphen (http://genetics.bwh.harvard.edu/pph/) and SIFT (http://sift.bii.a-star.edu.sg/). The allele frequencies were evaluated using the ALLELE and HAPLOTYPE procedures of SAS/Genetics, Version 9.3 (Statistical Analysis System Institute, Cary, NC, USA, 2012). Linkage disequilibrium among the *FGF5* polymorphisms was estimated using haploview, version 4.1 (Barrett *et al.* 2005). Intronic mutations were analysed using the Human Splicing Finder, version 2.4.1 (http://www.umd.be/HSF/).

### Results

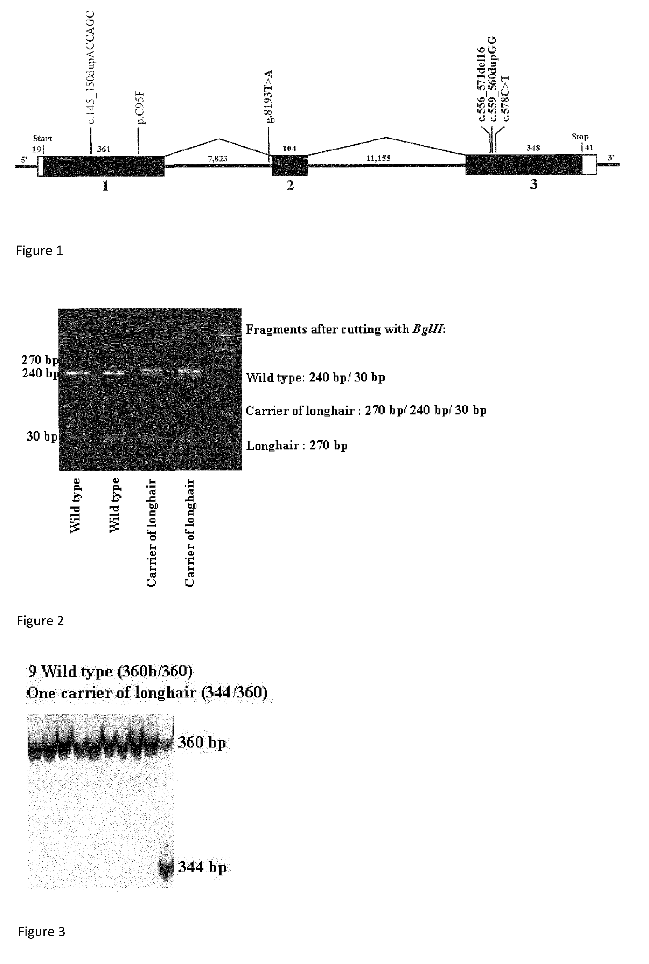

Sequencing of cDNA transcripts as well as all exons and their neighbouring intronic parts on genomic DNA revealed four novel mutations in *FGF5 (FGF5:g.8193T>A, FGF5:c.556_571del16, FGF5:c.559_560dupGG, FGF5:c.578C>T)* associated with the long hair phenotype (Table 1, figure 1).

The *FGF5:g.8193T>A* transversion was observed in three Afghan hounds, the *FGF5:c.556_571del16* deletion in one Eurasier, the FGF5;c.559_560dupGG duplication in twelve Afghan hounds and one Eurasier and the *FGF5:c.578C>T* transition was found in 14 Akitas, 26 Samoyeds and four Siberian huskies. Three of these four mutations *(FGF5:c.556-571del16, FGF5:c.559_560dupGG, FGF5:c.578C>T)* were located in the coding sequence of FGF5 and one mutation was located in intron 1 *(FGF5:g.8193T>A).* The novel exonic mutation *FGF5:c.578C>T* is predicted to change an amino acid (FGF5:p.Ala193Val). *FGF5:c.556_571del16* and *FGF5:c.559_560dupGG* result in a frameshift with a premature stop codon. The polymorphisms *FGF5:c.556_571del16* and *FGF5:c.559_560dupGG* lead to a very similar truncated protein (Figure 3). Furthermore, all exonic polymorphisms were located in regions of *FGF5* which are highly conserved among mammalian species. The intronic mutation *FGF5:g.8193T>A* in intron 1 in Afghan hounds was predicted to create a new 3' splice site, nine base pairs upstream of exon 2. However, analysis of cDNA showed no evidence for the acceptance of this new splice site but the existence of this new splice site seems to influence splicing of exon 2. We were able to show that this specific mutation in intron 1 prevents splicing of exon 2. The polymorphisms *FGF5:c.556_571del16, FGF5:c.559_560dupGG* and *FGF5:c.578C>T* were located in exon 3 downstream of the stop codon of FGF5 short form. Therefore, all newly detected mutations had only an effect on the FGF5 long variant.

Afghan hounds are phenotypically fixed for longhaired phenotypes and we detected in these dogs two novel mutations *(FGF5:g.8193T>A* and *FGF5:c.559_560dupGG)* besides the *FGF5:p.Cys95Phe* mutation.

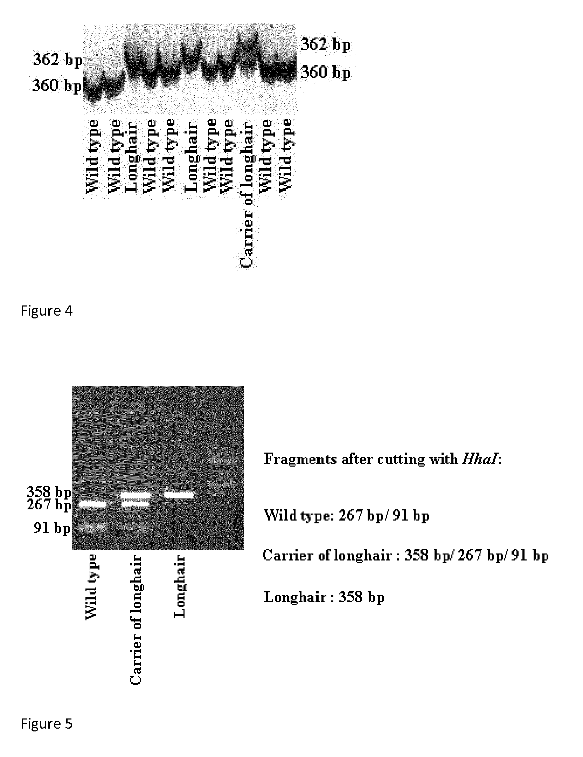

Two Afghan hounds were homozygous for the *FGF5:p.Cys95Phe* mutation, two other were homozygous for the *FGF5:c.559_560dupGG* duplication (see figure 4). The other twelve Afghan hounds analyzed in this study were compound heterozygous for the *FGF5:p.Cys95Phe* mutation and one of the other newly detected polymorphisms (Table 1, Figure 2). For one Afghan hound which was compound heterozygous for the *FGF5:g.8193T>A* and the *FGF5:c.559_560dupGG* mutation, we were able to demonstrate by cDNA analysis that only the shortened transcript variant 2 of FGF5 (FGF5 short form) was processed if the *FGF5:g.8193T>A* mutation was present. This compound heterozygous dog accumulates only a damaged transcript variant 1 of *FGF5 (FGF5:c.559_560dupGG).* We assumed that the other two Afghan hounds were compound heterozygous carriers of the *FGF5:g.8193T>A* and the FGF5:p.Cys95Phe mutations processed only one damaged transcript variant 1 (FGF5:p.Cys95Phe) and two different FGF5 short forms (FGF5 wild type and FGF5:p.Cys95Phe). Pedigree analysis revealed that two of the three Afghan hounds which carried the *FGF5:g.8193T>A* polymorphism were full sibs and these three dogs shared a common ancestor in the fifth generation. Afghan hounds with different mutations in *FGF5* showed a similar phenotype with regard to the hair length.

All ten longhaired Akitas were homozygous for the novel *FGF5:c.578C>T* mutation (Table 1 and Figure 5). This transition led to an amino acid substitution in a highly conserved region of exon 3 of *FGF5.* The long hair phenotype becomes obvious in three to four week old Akitas. The longhaired Akitas were also homozygous for a duplication *(FGF5c.145_150dupAGGAGG)* which plays no role in the development of long hair. None of the 6 shorthaired Akitas had two copies of any of the novel mutations described in this study, nor the previously described mutations which cause long hair.

Five of the seven longhaired Eurasiers were homozygous for the FGF5:p.Cys95Phe mutation while the other two Eurasiers were heterozygous for the FGF5:p.Cys95Phe mutation and either the *FGF5:c.556_571del16* or the *FGF5:c.559_560dupGG* mutation, respectively (Table 1). *FGF5:c.556_571del16* was only observed in one Eurasier among all dogs studied.

In the Samoyed only long hair phenotypes are observed. In Samoyeds, two *FGF5* mutations were frequently observed. Besides FGF5:p.Cys95Phe (homozygous in four dogs) the novel *FGF5:c.578C>T* mutation (homozygous in five dogs) was either homozygous or heterozygous. Most of the longhaired Samoyeds (21 dogs) were compound heterozygous for these mutations.

The previously discovered *FGF5c.14_150dupACCAGC* in exon 1 (Housley & Venta 2006) was present in several breeds of different hair length phenotypes including the Afghan hound, Akita, Alaskan malamute, shorthaired Dachshund, Dalmatian, Eurasier, German shepherd dog, German wirehaired pointer, Samoyed, and Siberian husky. All seven wolves included in this study were either heterozygous or homozygous for this duplication.

In order to clarify if dogs heterozygous for two or more mutations have inherited copies of these mutations by either parents or just one parent we used haplotype analysis and estimated linkage disequilibrium among all mutations found in FGF5. Haplotype analysis showed that the different mutations which led to long hair had been inherited independently and therefore originates from different parents. The duplication in exon 1 of *FGF5* which is not responsible for the long hair phenotype was in high linkage disequilibrium (LD) with the *FGF5:p.Cys95Phe* mutation (*r*² = 0.70) and the *FGF5:g.8193T>A SNP (r²* = 0.66). All other SNPs had a low LD *(r²* < 0.23) among each other.

## Claims

1. Process for the analysis of the genetic disposition of long hair phenotype in canidae wherein in at least one allele of the gene *FGF5* at least one of the mutations consisting of: *FGF5:c.578C>T, FGF5:c.559_560dupGG, FGF5:g.8193T>A* or *FGF5:c.556_571del16* is determined.

2. The process according to claim 1, wherein the mutation determined is *FGF5:c.578C>T.*

3. The process according to claim 1 or 2, wherein the mutation is present on both alleles.

4. The process according to any one of the preceding claims, wherein the genetic disposition is determined in a canidae selected from Akita, Samoyed, Afghan hound, Eurasier, and Siberian husky.

5. The process according to any one of the preceding claims, wherein mutation *FGF5:c.578C>T* is determined in Akita, Samoyed, and Siberian husky.

6. The process according to any one of the preceding claims for selecting an individual canidae for breeding.

7. The process according to any one of the preceding claims, wherein the mutation is determined on the coding strand or the non-coding strand.

8. The use of at least one of the mutations of *FGF5:c.578C>T, FGF5:c.559_560dupGG, FGF5:g.8193T>A* or *FGF5:c.556_571del16* for determining a genetic disposition for a long hair phenotype in a canidae.

9. Genetic marker for use in determining genetic disposition of canidae for a long hair phenotype by a process according to any one of claims 1 to 8, **characterized in that** the genetic marker is *FGF5:c.578C>T, FGF5:c.559_560dupGG, FGF5:g.8193T>A* or *FGF5:c.556_571del16.*

10. Test system or kit for use in determining genetic disposition of long hair phenotype in canidae comprising means for determining the presence of at least one mutation in at least one allele of the gene *FGF5,* said mutations are selected from the group consisting of: *FGF5:c.578C>T, FGF5:c.559_560dupGG, FGF5:g.8193T>A* or *FGF5:c.556_571del16,* and instructions for use said means in a process according to any one of claims 1 to 8.

11. Isolated nucleic acid molecule comprising a mutation in the gene *FGF5* selected from *FGF5:c.578C>T, FGF5:c.559_560dupGG, FGF5:g.8193T>A* or *FGF5:c.556_571del16.*

12. Isolated Oligonucleotide or probe comprising a nucleic acid sequence of the gene *FGF5* having a mutation of the group consisting of *FGF5:c.578C>T, FGF5:c.559_560dupGG, FGF5:g.8193T>A* or *FGF5:c.556_571del16.*


SEQUENCE LISTING

<110>  Stiftung Tieraerztliche Hochschule Hannover

<120>  Long hair phenotype in canidae

<130>  3064-003-EP-1

<160>  10

<170>  PatentIn version 3.5

<210>  1
<211>  20
<212>  DNA
<213>  artificial

<220>
<223>  synthetic oligonucleotide

<400>  1
cctcttcttc ctccgtctcc                                                    20


<210>  2
<211>  18
<212>  DNA
<213>  artificial

<220>
<223>  synthetic oligonucleotide

<400>  2
ccagggtgca aaacaacc                                                      18


<210>  3
<211>  30
<212>  DNA
<213>  artificial

<220>
<223>  synthetic oligonucleotide

<400>  3
agaggagtct gtgttttatt ttgggagatc                                         30


<210>  4
<211>  24
<212>  DNA
<213>  artificial

<220>
<223>  synthetic oligonucleotide

<400>  4
cccaagaaat cattaagtcc tctg                                               24


<210>  5
<211>  20
<212>  DNA
<213>  artificial

<220>
<223>  synthetic olionucleotide

<400>  5
gttcagggag cgatttcaag                                                   20


<210>  6
<211>  19
<212>  DNA
<213>  artificial

<220>
<223>  synthetic oligonucleotide

<400>  6
gcaaggcatg gtttctcac                                                    19


<210>  7
<211>  20
<212>  DNA
<213>  artificial

<220>
<223>  synthetic oligonucleotide

<400>  7
gttcagggag cgatttcaag                                                   20


<210>  8
<211>  19
<212>  DNA
<213>  artificial

<220>
<223>  synthetic oligonucleotide

<400>  8
gcaaggcatg gtttctcac                                                    19


<210>  9
<211>  20
<212>  DNA
<213>  artificial

<220>
<223>  synthetic oligonucleotide

<400>  9
gttcagggag cgatttcaag                                                   20


<210>  10
<211>  20
<212>  DNA
<213>  artificial

<220>
<223>  synthetic oligonucleotide

<400>  10
aaggcatggt ttctcaccag                                                   20